# EUROPEAN PATENT APPLICATION

(11) **EP 3 363 392 A1**
(43) Date of publication of application: **22.08.2018**
(21) Application number: 18156394.1
(22) Date of filing: 13.02.2018
(51) Int. Cl.: A61B 17/34, A61B 90/11

(54) **MEDICAL GUIDANCE DEVICE WITH CLOSED-FRAME ARC GUIDE**

(30) Priority: 16.02.2017 US 201762460025 P; 09.11.2017 US 201715808703
(71) Applicant: Canon U.S.A. Inc., Melville, New York 11747 (US)
(72) Inventor: KATO, Takahisa, Irvine,, CA California 92618 (US); HYDRICK, Luke, Boston,, MA Massachusetts 02210 (US); BOGNER, Eric, Boston,, MA Massachusetts 02210 (US); DANIELS, Barret, Irvine,, CA California 92618 (US); NAKAMURA, Hitoshi, Irvine,, CA California 92618 (US); DESAI, Devashree, Ashland,, MA Massachusetts 01721 (US); PEREGO, Federico, Boston,, MA Massachusetts 02210 (US)
(74) Representative: TBK

(57) **Abstract**

Medical guidance device capable of improving precision and accuracy of medical surgery by reducing human error involved in the insertion of a medical tool into a patient, via a closed-frame arc guide.

## Description

### FIELD OF THE DISCLOSURE

The present disclosure relates generally to apparatus and methods for medical guidance device and, more particularly, to a medical guidance device containing a closed-frame arc guide.

### BACKGROUND OF THE DISCLOSURE

Accurate and precise manipulation and orientation of medical tools is a critical factor in the successful use of these tools in surgical procedures. In particular, the accurate and precise orientation of a medical tool is especially critical in the insertion of a needle-like tool according to plan based on medical images, such as computed tomography (CT) and Magnetic Resonance Imaging (MRI) in percutaneous interventions.

In addition, intuitive understanding of the orientation of the medical tools palliates difficulty of the surgical procedures.

In order to achieve an accurate and intuitive orientation of the medical tools, and to reduce its user dependency, different systems and methodologies have been disclosed.

By way of example, United States Patent No. 9,408,627 (hereafter "'627 patent") discloses a whole-body stereotactic needle placement device. The device has a circular base plate, semicircular rotating arc that rotates on the base plate, a needle guide that guides the needle placed in a needle sizing tube, a needle guide cover and a locking pin that locks the needle guide cover to the needle guide. Furthermore, United States Patent No. 9,125,676 (hereafter "'676 patent") discloses a similar whole-body stereotactic needle placement device with a locking pin to lock the semicircular rotating arc perpendicular to the circular base plate.

However, the existing art suffers from a multitude of drawbacks which limit and hinder the use of medical devices. For instance, the semicircular arc guides of the devices detailed in the '627 patent and '676 patent are a cantilever shape with two ends, and are attached to the circular base plate or the supporting ring with these two ends. The cantilever shape is compliant against induced forces at both ends, and may experience large deformation from these induced forces at both ends when these guides are assembled. Therefore, the semicircular arc guides in '627 patent and '676 patent may include assembly error and relatively low position accuracy when these guides are attached on the circular base plate or the supporting ring. This assembly error also involves deviation of position accuracy among individual devices and different assembly incidents.

Moreover, since these two ends are faced with different parts, cramping these two ends may include relatively low stiffness and room to dislocate. Therefore, the semicircular arc guides cannot maintain a guiding position precisely when they are subjected to the force from the medical tool that the semicircular arc guides are guiding. Also, when the semicircular arc guides have residual stress by manufacturing, the residual stress can deteriorate position accuracy of the guide because of dislocation of these two ends.

In addition, the semicircular arc guides in the '676 patent are locked and kept perpendicular to the circular base plate and the supporting ring by using the locking pin. An assembly with this locking pin may include relatively large angle error because the fixing position with the locking pin is close to the triangle vertex of the angle and increases an angular error with the small position error. At the same time, the semicircular arc guide is free to rotate to this angular-error direction when the semicircular arc guide is unlocked without the locking pin. Therefore, the semicircular arc guide in '676 patent may include large angular error and large deviation of the angular error in every assembly incident.

Furthermore, the locking pin and the fixing screws are small parts that users are forced to handle in a surgical area. These parts are potentially lost and may bother fluent progress of an intervention.

As for the '627 patent, the circular base plate exposes the ring ridge, which is the bearing surface for the semicircular arc guide, to an external environment. In this configuration, the bearing surface is at risk of amassing dusts and fluid, from the external environment, during the intervention.

Accordingly, it is particularly beneficial to disclose a medical guidance device and method addressing the shortcomings in the prior art, and advancing the technology to yield a safer more accurate and precise apparatus and accompanying method of utilizing the apparatus.

### SUMMARY

The present disclosure teaches a medical guidance device incorporating a very rigid guide by using the needle guide with the arc shape guide and a closed-frame structure. Accordingly, the medical guidance device can guide a medical tool to the target position along the planned trajectory in a precise and consistent manner.

In various embodiments, the needle guide can be attached and detached from the movable structure even after the medical tool is inserted by using the space in the first part of the circumference. By detaching the needle guide, the physician can use the main opening to give necessary treatments to the patient and can avoid a potential obstacle for the inserted needle without dislocate rest of the device. Besides that, the medical guidance device has only one mechanical interface between the movable ring and the needle guide, and can provide almost minimal assembly steps to detach and attach the needle guide. Accordingly, the medical guidance device can increase efficiency of the intervention and decrease position errors and human factor errors caused by assembly.

In yet other embodiments, the needle guidance device houses the bearing part for the movable ring on the base plate. Therefore, the bearing part can maintain a clean surface during the intervention and the storage by avoiding dusts and liquid from an external environment.

In other embodiments of the subject disclosure, the medical guidance device may utilize an adhesive layer with the center marker to minimize mounting errors of the device by aligning the position of the rotation axis of the movable ring to a skin entry point that physicians planed. Accordingly, the adhesive layer can reduce the number of actions necessary to apply the device to the patient. Therefore, the device can reduce duration of the intervention.

In yet other embodiments, the subject medical guidance device, by having the adhesive layer on the bottom surface, can reduce the footprint in comparison to the case of taping the device down or of screwing the device down to the patient. Therefore, the device can provide a wider surgical space and can improve usability and visibility.

In other embodiment, the implementation of a peel-away part in the adhesive layer allows the physician to apply the device with the center marker first, and make the main opening second. Therefore, the device can achieve both precise mounting position and a wide opening for the intervention with minimal steps.

In various embodiment of the subject disclosure, the inclusion of different colors on the first part of the circumference or on the guidance surface from the other part can reduce the human-factor error to mix the guidance surface up with the opposite surface in the arc shape guide.

In other embodiments, the subject guidance device may incorporate angular reference marks on the arc shape guide to provide precise angle information for physicians. By combining the angular reference marks and the guide surface, the physicians can determine an insertion trajectory of the medical tool in three-dimensional space. Also, the angular reference marks are visible in the medical images that physicians use to guide the medical tool. The physician can compare the planned trajectory or the inserted medical tool with the angular reference marks in the medical images and determine a target angular marker in the medical images. Therefore, the medical guidance device can improve insertion accuracy and its deviation among different individual skills and experiences.

Accordingly, by making the guide surface visible in the medical images, the physician can find the guide surface in the medical images intuitively and quickly. Because the planned trajectory or the inserted medical tool locates close to the guide surface, the physician can evaluate and determine a new trajectory in the images by comparing the guide surface in the medical images with the planned trajectory or the inserted medical tool.

In yet another embodiment, the physician can find a cross sectional image that includes angular reference markers intuitively and quickly. Therefore, the physician can use the angular reference markers in the medical images intuitively and quickly.

In various embodiments, the angular reference markers may be made by luminous bodies, resulting in the angular reference markers increasing visibility in the various lighting environment in the operational room. By way of example, when the angular reference markers are LED array, the medical guidance device can light only the angular reference marker that physician may be interested in. Therefore, the medical guidance device increase efficiency of the intervention, and reduces human-factor error and mental stress to use the medical guidance device.

In various embodiments, the medical guidance device may incorporate real-time positioning of the movable ring with the rotary encoder, allowing the physician to position the movable ring precisely. Accordingly, the medical guidance device can reduce human-factor error and mental stress to adjust the movable ring to the planned position.

In other contemplated embodiment, by housing the rotary encoder with the base plate and the movable ring, the rotary encoder and its scale can maintain the gap between them clean from contaminations from the external environment. As such, the medial guidance device can increase robustness and stability of the device operation. Specifically, because the base plate is close to the area for the intervention, this function is important to use the medical guidance device in the intervention.

In yet additional embodiments, the medical guidance device includes an indicator with the microcontroller which can reflect the real-time position of the movable ring interactively. Therefore, the medical guidance device can further reduce human-factor error and mental stress to adjust the movable ring to the planned position. Furthermore, by separating the controller box from the parts mounted on the patient, the medical guidance device can reduce electronics to be exposed in the imaging field. Therefore, the medical guidance device can reduce deterioration of the image quality. Moreover, by reducing electronics from the parts mounted on the patient, the medical guidance device can reduce the electronics that need to be sterilized. Also, the medical guidance device can decrease the footprint of the parts mounted on the patient, and can provide a wider area of the intervention for the physician.

These and other objects, features, and advantages of the present disclosure will become apparent upon reading the following detailed description of exemplary embodiments of the present disclosure, when taken in conjunction with the appended drawings, and provided paragraphs.

### BRIEF DESCRIPTION OF THE DRAWINGS

Further objects, features and advantages of the present invention will become apparent from the following detailed description when taken in conjunction with the accompanying figures showing illustrative embodiments of the present invention.
Fig. 1(a) is a top view of a medical guidance device, according to one or more embodiments of the present subject matter.
Fig. 1(b) is a cross sectional view at lines A-A of Fig. 1 (a), according to one or more embodiments of the present subject matter.
Fig. 1(c) is a cross sectional view at lines B-B of Fig. 1(a), according to one or more embodiments of the present subject matter.
Fig. 2(a) is a top view of a medical guidance device, according to one or more embodiments of the present subject matter.
Fig. 2(b) is a cross sectional views at A-A lines of Fig. 2(a), according to one or more embodiments of the present subject matter.
Fig. 2(c) is a cross sectional views at B-B lines of Fig. 2(a), according to one or more embodiments of the present subject matter.
Fig. 3(a) is a perspective view of an assembled medical guidance device, according to one or more embodiments of the present subject matter.
Fig. 3(b) is an exploded view of the assembled medical guidance device provided in Fig. 3(a), according to one or more embodiments of the present subject matter.
Fig. 3(c) is a detailed view of a portion of the assembled medical guidance device provided in Fig. 3(a), according to one or more embodiments of the present subject matter.
Figs. 4(a) through 4(d) provide various designs of one or more components of a medical guidance device, according to one or more embodiments of the present subject matter.
Fig. 5 provides a schematic system diagram of a medical guidance device including a controller box, according to one or more embodiments of the present subject matter.

Throughout the Figures, the same reference numerals and characters, unless otherwise stated, are used to denote like features, elements, components or portions of the illustrated embodiments. In addition, reference numeral(s) including by the designation "'" (e.g. 12' or 24') signify secondary elements and/or references of the same nature and/or kind. Moreover, while the subject disclosure will now be described in detail with reference to the Figures, it is done so in connection with the illustrative embodiments. It is intended that changes and modifications can be made to the described embodiments without departing from the true scope and spirit of the subject disclosure as defined by the appended paragraphs.

### DETAILED DESCRIPTION OF THE DISCLOSURE

The present disclosure details a medical device capable of being steered for guidance through passages. More specifically, the subject medical device contains a cavity for accepting medical tools and devices including endoscopes, cameras, and catheters, and the ability to guide or maneuver the medical tool or device through passages.

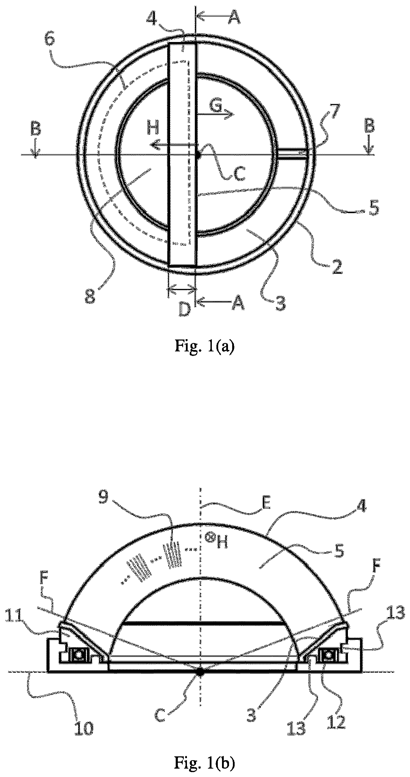

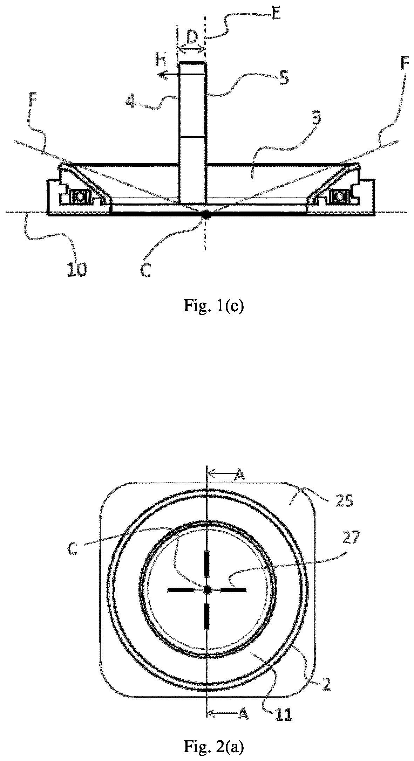

Figs. 1(a) through 1(c) provide views of a medical guidance device 1, according to one or more embodiments of the present subject matter, including cross-sectional views detailed in Fig. 1(b) and 1(c). The medical guidance device 1 comprises a base plate 2, a movable ring 11, a detachable guide 3, an arc shape guide 4 and a main opening 8. The base plate 2 is mounted on the patient at mounting surface 10. The base plate 2 also has a ring shape and is engaged with the movable ring 11 (Fig. 1(b) and (c)). The movable ring 11 is rotatable around the axis E with the bearing 12 on the base plate 2. The axis E passes point C on the mounting surface 10. Also, the base plate 2 and the movable ring 11 have seal structures 13, which protect the bearing 12 from contamination in an external environment.

The movable ring 11 also includes mechanical interface 18 (not shown in Fig. 1). With the mechanical interface 18, the ring frame 3 is engaged with the movable ring 11. The ring frame 3 is monolithically made with the arc shape guide 4, and includes space 7 to release medical tool 22 (not shown in Fig. 1). The arc shape guide 4 is monolithically attached on the ring frame 3, and brides on the ring frame 3 over the main opening 8. The arc shape guide 4 also includes the guide surface 5 and angular reference marks 9. The guide surface 5 includes axis E and have the thickness D from the guide surface 5 to create a guidance area toward arrow G. The angular reference marks 9 are line marks to signify an angle around point C on the guide surface 5. By rotating the movable ring 11 around axis E, the angular reference marks 9 can also rotate around axis E with the guide surface 5. By using the angular reference marks 9 and the movable ring 11, the medical guidance device 1 can localize fine grids of remote center of motion with point C, which are cone shape grids with generator F along the point C as a pivot. The remote center of motion is a model of physician's maneuver of the medical tool. The point C can be aligned to a skin entry point of the medical tool, which is defined by considering obstacles close to the skin. With the fixed point C, the physician can select intended trajectory to the target by using an appropriate position of the movable ring 11 and the angular reference marks 9. After determining the position of movable ring 11 and the angular reference marks 9, the physician can insert the medical tool with guidance the guide surface 5 at the target angular reference mark 9.

When the guide surface 5 guides the medical tool, the arc shape guide 4 will be subjected to force to arrow H. The force to arrow H is supported by closed frame 6, which comprises arc shape guide 4 and half of the ring frame 3 at the opposite side of the guide area. In comparison to a cantilever shape, i.e. an open frame, the closed frame 6 has larger stiffness and can cope with the force to arrow H with smaller deflection. Also stiffness of the closed frame 6 can increase by increasing thickness D while keeping the opening in the guidance area.

After the medical tool is inserted, the ring frame 3 and the arc shape guide 4 can be detached from the movable ring 11. The medical tool can go through the space 7. Therefore, the ring frame 3 can the arc shape guide 4 can be detached even when the medical tool is tethered, for instance percutaneous ablation probes.

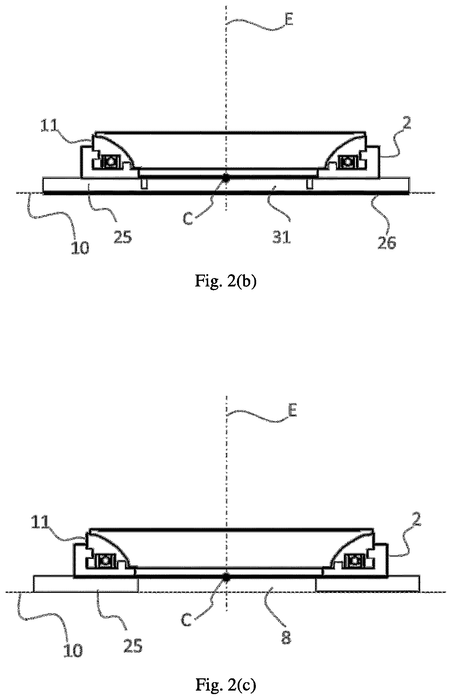

Figs. 2(a) through 2(c) provide various views of a medical guidance device 1, according to one or more embodiments of the present subject matter, including cross-sectional views detailed in Fig. 2(b) and 2(c). Components similar to those detailed in Figs. 1(a) through 1(c) are denoted by the same reference numerals, and accordingly, the descriptions provided above are incorporated by reference and not repeated.

Varying features different from the embodiment presented in Figs. 1(a) through 1(c), include an adhesive layer 25. The adhesive layer 25 is transparent and is attached on the base plate 2 and includes the mounting surface 10 on the bottom surface of the adhesive layer 25. This bottom surface is sticky surface 26 and can fix the base plate 2 on the patient. On the adhesive layer 25, the center marker 27 is printed and signifies point C with cross symbol. By using the center marker 27, the physician can easily align point C to the skin entry point on the patient. After mounting the medical guidance device 1, peel-away part 31 is removed and creates a main opening 8. After removing the peel-away part 31, rest of the adhesive layer 25 keep fixing the base plate 10 on the patient.

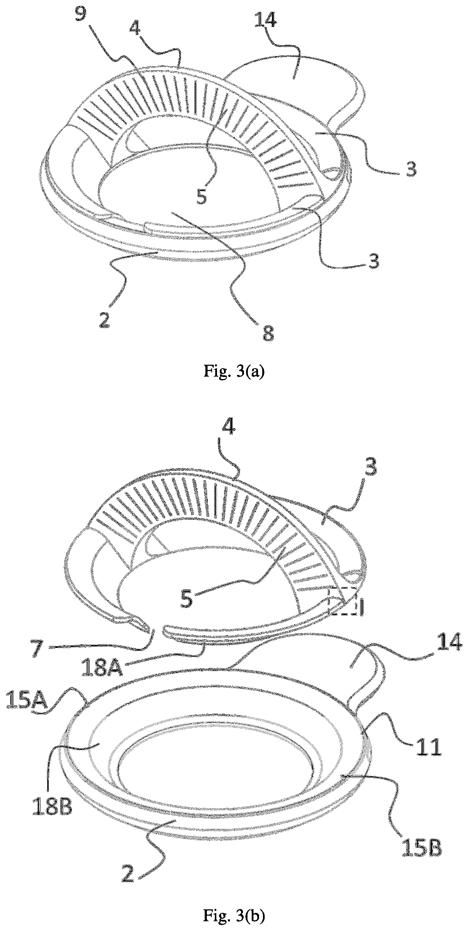

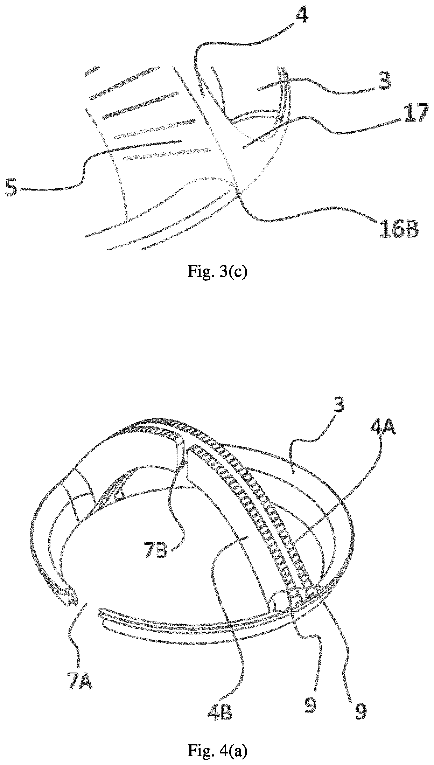

Figs. 3(a) through 3(c) provide views of an assembled medical guidance device, according to one or more embodiments of the present subject matter, including an exploded view in Fig. 3(b), as well as a detailed view (Fig. 3(c)) of a portion of the assembled medical guidance device provided in Fig. 3(a)

As before, components similar to those of the previously disclosed embodiment are denoted by the same reference numerals, and descriptions provided above are incorporated by reference and not repeated.

As provided in Fig. 3 (a), the base plate 2 has an outer diameter of 80 mm. The diameter of the main opening 8 is 60 mm. The main opening 8 of 60 mm provides the physician with enough room for the intervention. Specifically, after the first medical tool is guided by the arc shape guide 4, the physician can detach the arc shape guide 4 with ring frame 3, and can insert a second medical tool in the main opening 8 without unmounting the base plate 2. The mechanical interface 18A and 18B is tapered. This tapered feature can align to mate the ring flame 3 to the base plate 2 in concentric configuration. Also, this tapered feature increase range of angles in angular reference marks 9 to sallower angle. Moreover, the tapered feature increases width of the cramping part of the arc shape guide 4 and makes the arc shape guide 4 rigid against the force from the medical tool (the force H in Fig. 1).

The mechanical interfaces 18A and 18B include keys 15A, 15B and keyways 16A, 16B. One of facets in the keys 15A and 15B is aligned to a plane position of the guide surface 5 in case of attaching the arc shape guide 4. Also, keyways 16A, 16B include a part of the guide surface 5 (Fig. 3 (c)). Therefore, by engaging keys 15A, 15B with keyways 16A, 16B, and the arc shape guide 4 can be aligned to designed position accurately and precisely. The arc shape guide 4 has fillet 17 only on the opposite side of the guide surface 5. This fillet can increase stiffness against the force from the medical tool (the force H in Fig. 1) and increase position accuracy of the guide surface 5 during insertion of the medical tool.

The base plate 2 also includes grip 14. The grip 14 provides the physician with holding place and can increase stability of the base plate during insertion of the medical tool. Moreover, the grip 14 can include electronics and visible markers for the medical images like CT and MRI, while minimizing a footprint of the base plate 2. The angular reference marks 9 in this embodiment are made of radio-opaque material. Therefore, the angular reference marks 9 are visible optically as well as in CT and X-ray images. The radio-opaque material can be the plastic including fillers of Barium Sulfate, bismuth subcarbonate, bismuth oxychloride, tungsten. Moreover, the guide surface 5 can be visible in CT and X-ray images with different contrast radiopaque plastic.

Additional embodiment will now be described with reference to Figs. 4(a) through 4(d), which provide various designs of one or more components of a medical guidance device, according to one or more embodiments of the present subject matter. Once again, components similar to those of the previously disclosed embodiments are denoted by the same reference numerals, and descriptions thereof are incorporated by reference.

The arc shape guide 4 in Fig. 4(a) includes two arc shape guides 4A and 4B. Also, the guidance surface 5 is a middle plane between the arc shape guides 4A and 4B. Also, the arc shape guide 4B includes space 7B. The gap between the arc shape guides 4A and 4B are slightly larger than the diameter of the medical tool intended to be used. The middle plane can be easily found by the eye by referring the spaces to both sides toward the arc shape guides 4A and 4B. The angular reference marks 9 are printed on the top surface of two arc shape guides 4A and 4B.

By signifying the guidance surface 5 with two separated physical surface of the arc shape guides 4A and 4B, the medical tool doesn't need to touch the parts. Therefore, the physician can insert the medial tool with superior tactile feedback. Also, with adjustment spaces in direction to the two arc shape guides 4A, 4B, the physician can easily adjust the medical tool to be on the guide surface 5.

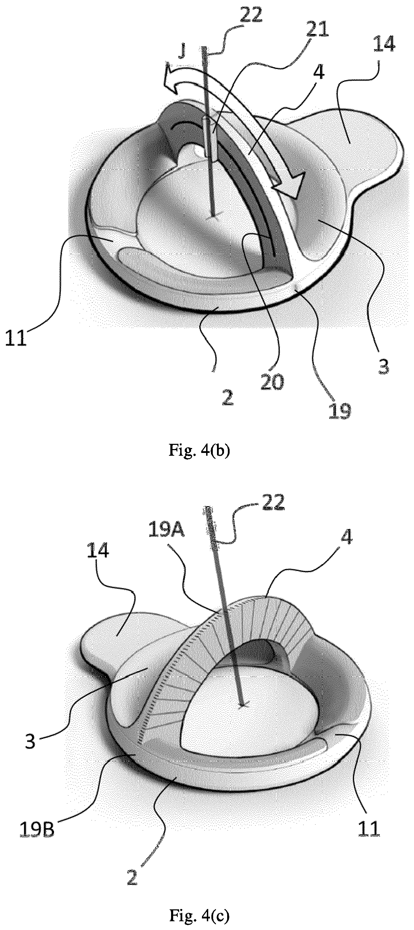

The arc shape guide 4 in Fig. 4(b) further comprises arc rail 20, needle guide 21. The needle guide 21 is slidable along the arc rail 20 toward arrow J. The needle guide 21 has a half cylindrical groove and can fits the medical tool 22. The needle guide 21 provides constrained guide for the medical tool 22. The needle guide 21 can accurately guide the medical tool 22 by directing the half cylindrical groove to the target trajectory. Specifically, when the target position is more deterministic, the needle guide 21 can increase accuracy and can reduce duration of the intervention effectively.

The medical guidance device provided in in Fig. 4(c) includes indicators 19A and 19B along circumference of the base plate 2 and the arc shape guide 4. The indicators 19A and 19B are LED array and are electrically driven by the electronics in grip 14. The indicators 19A and 19B can inform user about the target position by lighting the closest LED to the target position. Because the physician doesn't need to read the angular reference marks 9, the indicators 19A and 19B can reduce duration of the intervention as well as mental stress of the physician.

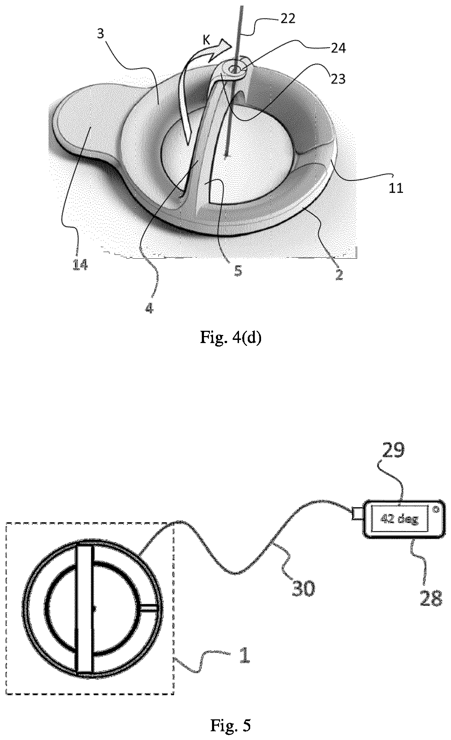

The arc shape guide 4 in Fig. 4(d) includes needle holder 23. The needle holder 23 has a similar arc rail structure on the opposite side of the guide surface 5 and slidable along the arc shape guide 4. Also, the needle holder 23 is retractable from the guide area, and attachable to the guide area along arrow K in the Fig. 4(d). The needle holder 23 includes through-hole and slit 24. The through-hole is large enough to loosely hold the medical tool 22. The needle holder 23 can accept the medical tool 22 at this thorough-hole through the slit 24 after the medial tool 22 is inserted. With the needle holder 23, the medical tool 22 can move freely within the thorough-hole, but won't fall down. Therefore, the needle holder 23 can improve handling management of the inserted medical tool 22 until the medical tool 22 ends the intervention.

Fig. 5 provides a schematic system diagram of a medical guidance device including a controller box, according to one or more embodiments of the present subject matter. As before, components similar to those of the previously disclosed embodiments are denoted by the same reference numerals, as such, prior descriptions thereof are incorporated herein, in their entirety, by reference.

Fig. 5 is a schematic system diagram of the medical guidance device 1 including a controller box 28. The medical guidance device 1 includes a rotary encoder (not shown in figure) and its sensor circuit board. The rotary encoder is mounted on the base plate 2 and is housed by the base plate 2 and the movable ring 11. The movable ring 11 includes a scale for the rotary encoder. The rotary encoder can measure rotational position of the movable ring 11. The controller box 28 includes indicator 29, a microcontroller and a battery (not shown in Fig. 5), and is electrically connected to the medical guidance device 1 by electric cable 30. The microcontroller communicates to the sensor circuit board. The battery is powering indicator 29, the rotary encoder, the sensor circuit board and the microcontroller. The indicator 29 can reflect the real-time rotational position of the movable ring 11 with digital indicator.

Medical guidance device capable of improving precision and accuracy of medical surgery by reducing human error involved in the insertion of a medical tool into a patient, via a closed-frame arc guide.

## Claims

1. A medical guidance device to guide a medical instrument comprising:
a base plate including a base-plate opening, and a bottom surface mounted to a patient;
a movable ring that rotatably attaches to the base plate, including a movable-ring opening aligned to the base-plate opening to form a main opening to access to the patient;
a needle guide including a ring frame and an arc shape guide, wherein the arc shape guide attaches to the ring frame at two ends of the arc shape guide and including a guide surface to provide a reference plane to guide the medical instrument, the ring frame detachably attached to the movable ring in a concentric fashion so that the guidance surface is rotatable with the movable ring against the base plate, wherein:
the ring frame includes a first part and second part of the circumference divided by two ends of the arc shape guide;
the first part of the circumference is facing the guidance surface and includes a space to release the medical instrument; and
the second part of the circumference forms a mechanical closed loop with the arc shape guide.

2. The medical guidance device according to claim 1, wherein the bottom surface mounted to the patient includes an adhesive layer with a sticky surface facing toward a direction of the patent, wherein the adhesive layer includes a center marker to represent a position of a rotation axis of the movable ring on the bottom surface.

3. The medical guidance device according to claim 2, wherein the adhesive layer includes a peel-away part to make the main opening when it is peeled away.

4. The medical guidance device according to claim 1, wherein either the first part of the circumference or the guidance surface has a different color from the other part.

5. The medical guidance device according to claim 1 further comprising:
an angular reference mark on the arc shape guide, wherein the angular reference mark signifies an angle around the position of the rotation axis of the movable ring at the bottom surface and on a plane including the guide surface, wherein the angular reference mark is radio opaque or visible in MRI images.

6. The medical guidance device according to claim 5, wherein the guide surface is radio opaque or visible in MRI images.

7. The medical guidance device according to claim 5, wherein the angular reference marks are luminous bodies.

8. The medical guidance device according to claim 1 further comprising:
a rotary encoder to measure a position of the movable ring interactively; and
a circuit board to process the signals from the rotary encoder, wherein the rotary encoder and the circuit board are on the base plate and are covered at least partially by the movable ring.

9. The medical guidance device according to claim 8 further comprising:
an indicator to indicate a real-time status of the device;
a microcontroller to process the information from the encoder and command to the indicator and the encoder; and
a controller box to include the microcontroller, wherein the microcontroller is electrically connected to the indicator and encoder; and the controller box is independent from the base plate, the movable ring and the needle guide.
